# EUROPEAN PATENT APPLICATION

(11) **EP 4 751 762 A1**
(43) Date of publication of application: **03.06.2026**
(21) Application number: 24215803.8
(22) Date of filing: 27.11.2024
(51) Int. Cl.: A61N 1/04, A61B 5/266, A61N 1/39

(54) **DEFIBRILLATOR ELECTRODE WITH ELECTROLYTE**

(71) Applicant: Koninklijke Philips N.V., 5656 AE Eindhoven (NL)
(72) Inventor: ROY, Lorne, 5656 AG Eindhoven (NL); JORGENSON, Dawn Blilie, 5656 AG Eindhoven (NL)
(74) Representative: Philips Intellectual Property & Standards

(57) **Abstract**

A system employing an external defibrillator (60), and a pair of defibrillator electrodes (30), each defibrillation electrode including an electrolyte bladder (50) adjoined to a conductive electrode pad (40). In operation, the electrolyte bladder (50)s release an electrolyte fluid from the electrolyte bladder (50)s as the conductive electrode pad (40)s are being attached to a permeable material covering a body of the patient to electrically couple the conductive electrode pad (40)s to the body of the patient. Upon the conductive electrode pad (40)s being electrically coupled to the body of the patient by the electrolyte fluids, the defibrillation electrodes are utilized to transmit electrical signals from a heart of the patient through the electrolyte fluid and the conductive electrode pad (40)s to the external defibrillator (60) and to deliver a defibrillation shock from the external defibrillator (60) through the conductive electrode pad (40)s and the electrolyte fluid to the heart of the patient.

## Description

### FIELD OF THE INVENTION

The present disclosure generally relates to defibrillator electrodes. The present disclosure particularly relates to an electrolyte enhancement of defibrillator electrodes.

### BACKGROUND OF THE INVENTION

External defibrillators as known in the art of the present disclosure employ defibrillator electrodes attached to skin of a patient experiencing cardia arrest whereby the defibrillator electrode creates an electrical pathway within a body of the patient for delivering a defibrillation shock to a heart of the patient. While time is of the essence to quickly and effectively attach the defibrillator electrodes to the skin of the patient to provide the electrotherapy to the patient, there nonetheless are numerous reasons that a resistance and/or an inability to timely attach the defibrillator electrodes can occur during the attachment process.

For example, cultural norms can lead to resistance to fully disrobe a patient to the extent necessary to apply defibrillation electrodes to the body of the patient. Also, excessive body hair on the body of the patient can prevent adequate skin contact for the defibrillator electrodes to effectively deliver electrotherapy, which can lead to a time-consuming need for shaving the body or re-applying the defibrillator electrodes.

The present disclosure addresses a need for defibrillator electrodes that can function despite obstructions to the body attachment process (e.g., chin clothing, bra straps, body hair, etc.).

### SUMMARY OF THE INVENTION

The present disclosure provides a deployment of an electrolytic fluid as defibrillator electrodes are applied to a body of a patient. The fluid becomes the conductive pathway for electrocardiogram and electrical therapy.

The present disclosure can be embodied as (1) a defibrillator electrode, (2) a defibrillation system, and (3) a defibrillation method.

Various embodiments of a defibrillator electrode of the present disclosure employ a conductive electrode pad and an electrolyte bladder adjoined to the conductive electrode pad.

When the conductive electrode pad is physically attached to a permeable material covering the body of the patient, the electrolyte bladder is configured to release an electrolyte fluid to electrically couple the conductive electrode pad to the body of the patient.

When the conductive electrode pad is electrically coupled to the body of a patient by the electrolyte fluid and when the conductive electrode pad is electrically connected to an external defibrillator, the conductive electrode pad is configured to transmit electrical signals from a heart of the patient to the external defibrillator and to deliver a defibrillation shock from the external defibrillator to the heart of the patient.

Various embodiments of a defibrillation system of the present disclosure employ an external defibrillator and a defibrillator electrode including an electrolyte bladder adjoined to a conductive electrode pad.

When the conductive electrode pad is physically attached to a permeable material covering the body of the patient, the electrolyte bladder is configured to release an electrolyte fluid to electrically couple the conductive electrode pad to the body of the patient.

When the conductive electrode pad is electrically coupled to the body of a patient by the electrolyte fluid and when the conductive electrode pad is electrically connected to an external defibrillator, the conductive electrode pad is configured to transmit electrical signals from a heart of the patient to the external defibrillator and to deliver a defibrillation shock from the external defibrillator to the heart of the patient.

Various embodiments of a defibrillation method of the present disclosure are executable by an external defibrillator and a pair of defibrillator electrode, each defibrillator electrode including an electrolyte bladder adjoined to a conductive electrode pad.

The defibrillation method involves a release of an electrolyte fluid from each electrolyte bladder as the respective conductive electrode pads are being attached to permeable material covering a body of the patient to electrically couple the conductive electrode pads to the body of the patient; and
upon the conductive electrode pads being electrically coupled to the body of the patient by the electrolyte fluid, transmitting electrical signals from a heart of the patient through electrolyte fluid and the defibrillator electrodes to the external defibrillator.

The foregoing exemplary embodiments and other embodiments of the present disclosure as well as various structures and advantages of the present disclosure will become further apparent to those having ordinary skill in the art from the following detailed description of various embodiments of the present disclosure read in conjunction with the accompanying drawings. The detailed description and drawings are merely illustrative of the present disclosure rather than limiting, the scope of the present disclosure being defined by the appended claims and equivalents thereof.

### BRIEF DESCRIPTION OF THE DRAWINGS

The present disclosure will present in detail the following description of exemplary embodiments with reference to the following figures wherein:
FIG. 1A illustrates an exemplary embodiment of a defibrillator electrode in the art of the present disclosure;
FIG. 1B illustrates an exemplary embodiment of a conductive electrode pad in accordance with the present disclosure;
FIG. 2 illustrates an exemplary embodiment of a defibrillation system in accordance with the present disclosure;
FIGS. 3A-3D illustrates an exemplary application of defibrillator electrodes of the present disclosure to a body of a patient; and
FIG. 4 illustrates an AED system in accordance with the present disclosure.

### DETAILED DESCRIPTION OF THE PREFERRED EMBODIMENTS

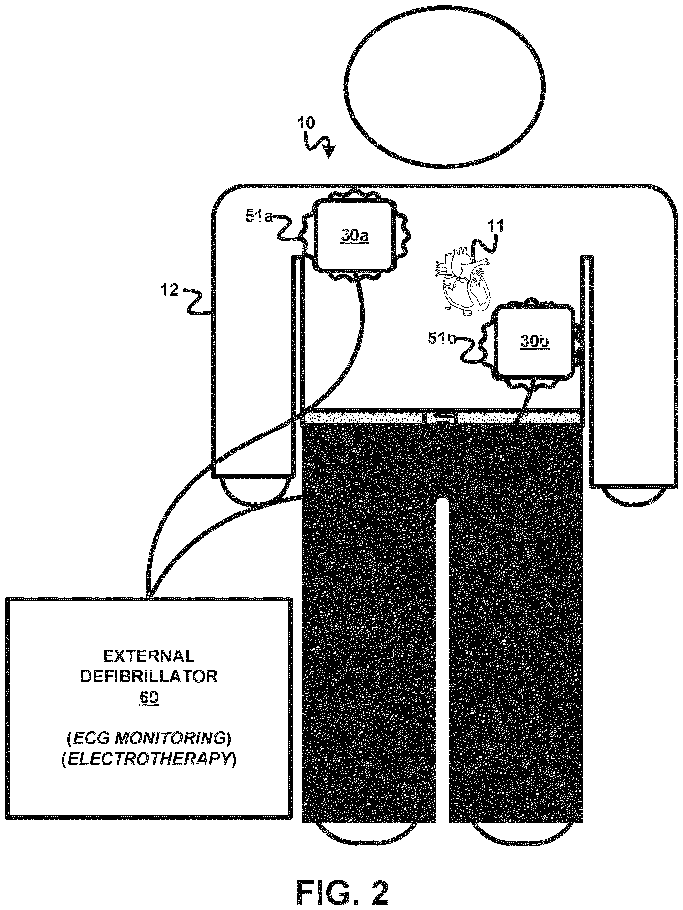

To facilitate an understanding of the present disclosure, the following description of FIGS. 2-4 describes and teaches exemplary embodiments of devices, systems and methods in accordance with the present disclosure. From the description of FIGS. 2-4, those having ordinary skill in the art of the present disclosure will appreciate how to apply the present disclosure to make and use additional embodiments of devices, systems and methods in accordance with the present disclosure.

Please note that the defibrillator electrodes as shown in the present disclosure are not drawn to scale, but to facilitate an understanding of the operating principles of the present disclosure.

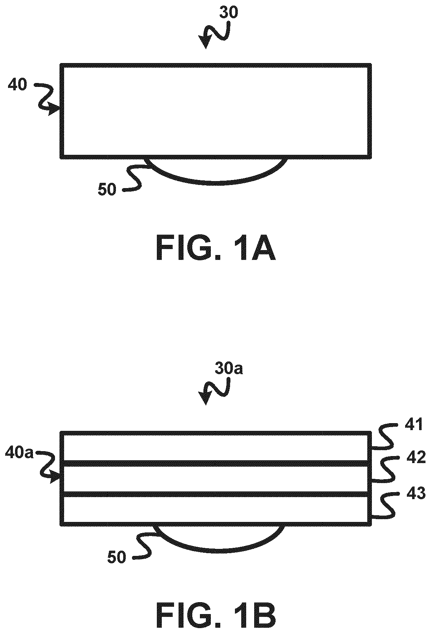

FIG. 1A illustrates a defibrillator electrode employing a conductive electrode pad 40 as known in the art of the present disclosure and an electrolyte bladder 50 in accordance with the present disclosure.

Referring to FIG. 2, in one exemplary embodiment, electrolyte bladder 50 is a skin containing an electrolyte until it is deployed by pressing down on pad 40 so the bladder 50 bursts releasing the electrolyte. In practice, the skin over the electrolyte would provide a seal to prevent exposure to air and drying out of the electrolyte for an extended shelf-life. The advantage of the electrolyte is that a thin clothing layer could remain on the patient because the electrolyte would penetrate and soak the clothing and provide an efficient transfer of energy for the defibrillation shock.

FIG. 1B illustrates an exemplary embodiment 40a of conductive electrode pad 40 (FIG. 1A).

Referring to FIG. 1B, conductive electrode pad 40a includes a flexible substrate 41 and an electrical conductor 42 having an outer surface that would face the subject. Electrical conductor 42 can a metal foil as known in the art of the present disclosure.

Pad 40a further includes a gel layer 43 covering at least a portion of the outer surface of electrical conductor 42 to aid in attaching the electrode to a skin of a subject and facilitating a good electrical contact when attached to skin. Electrolyte bladder 50 facilitates a good electric contact when gel layer 43 is contacting clothing.

FIG. 2 illustrates a defibrillation system of the present disclosure employing an external defibrillator 60 for executing electrogram monitoring and electrotherapy as known in the art of the present disclosure (e.g., an automatic external defibrillator, a semi-automatic defibrillator or a manual external defibrillator.

To this end, external defibrillator is electrically connected to defibrillator electrodes 30a and 30b, which are electrically coupled to a body of patient 10 via electrolyte fluid soaked through a shirt 12.

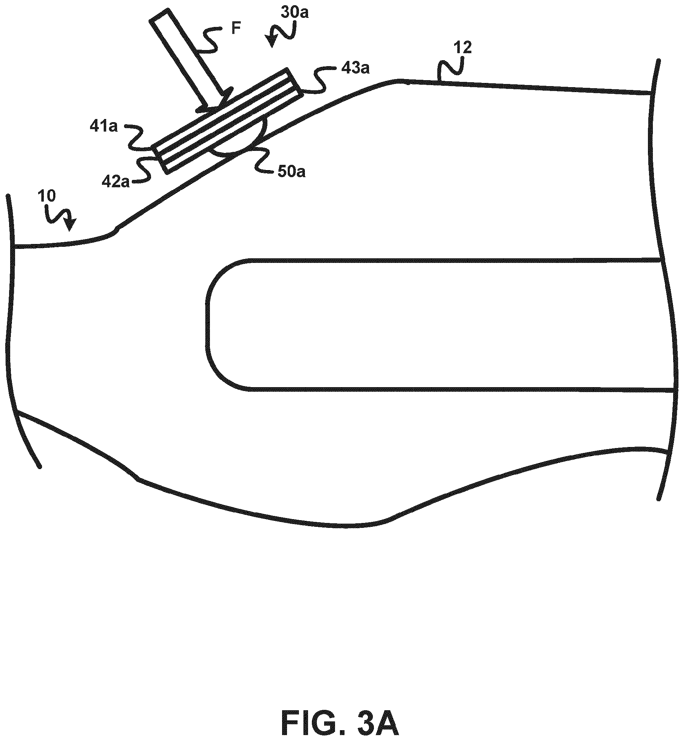

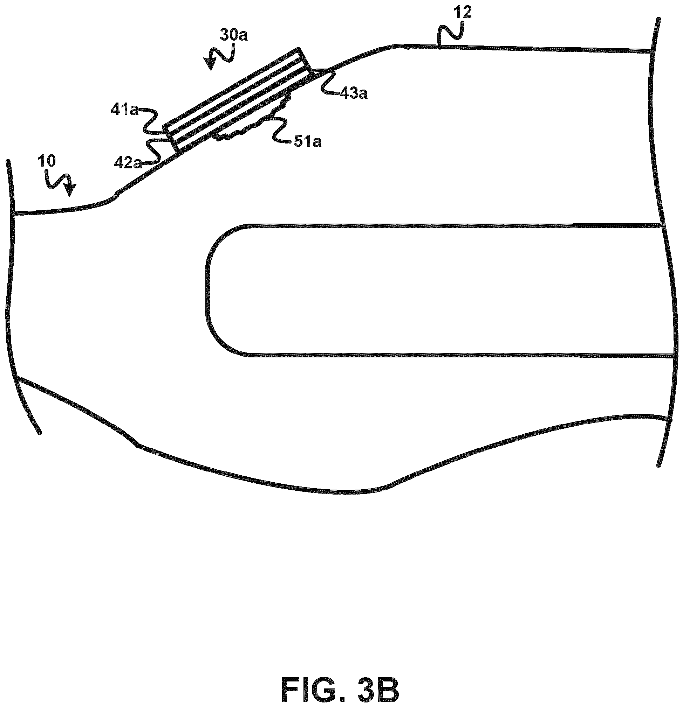

FIG. 3A illustrates defibrillator electrode 30a being positioned on shirt 12 at a sternum of patient 10 whereby a downward force F releases the electrolyte fluid 51a from electrolyte bladder 50a as shown in FIG. 3B.

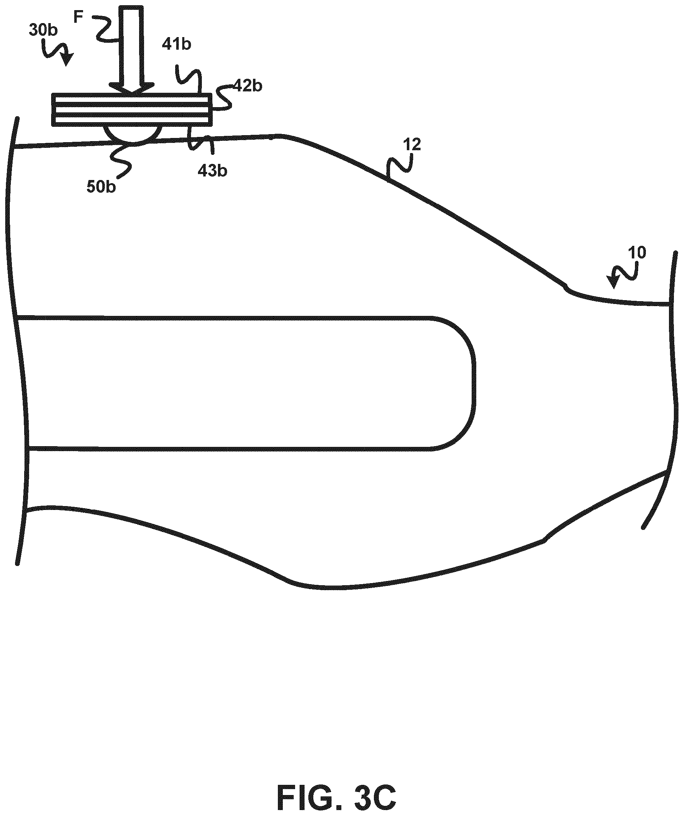

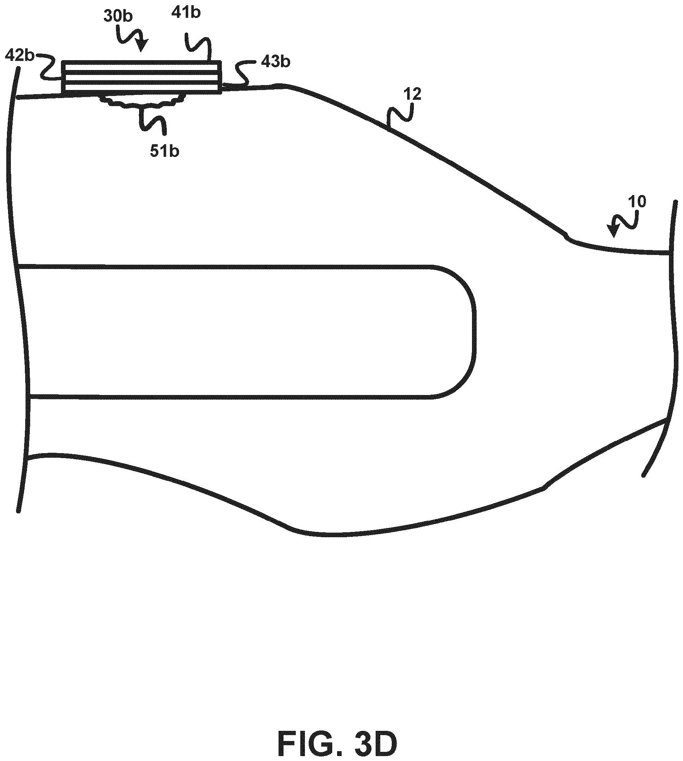

FIG. 3C illustrates a defibrillator electrode 30b being positioned on shirt 12 at a lateral position of patient 10 whereby a downward force F releases the electrolyte fluid 5 1b from electrolyte bladder 50b as shown in FIG. 3D.

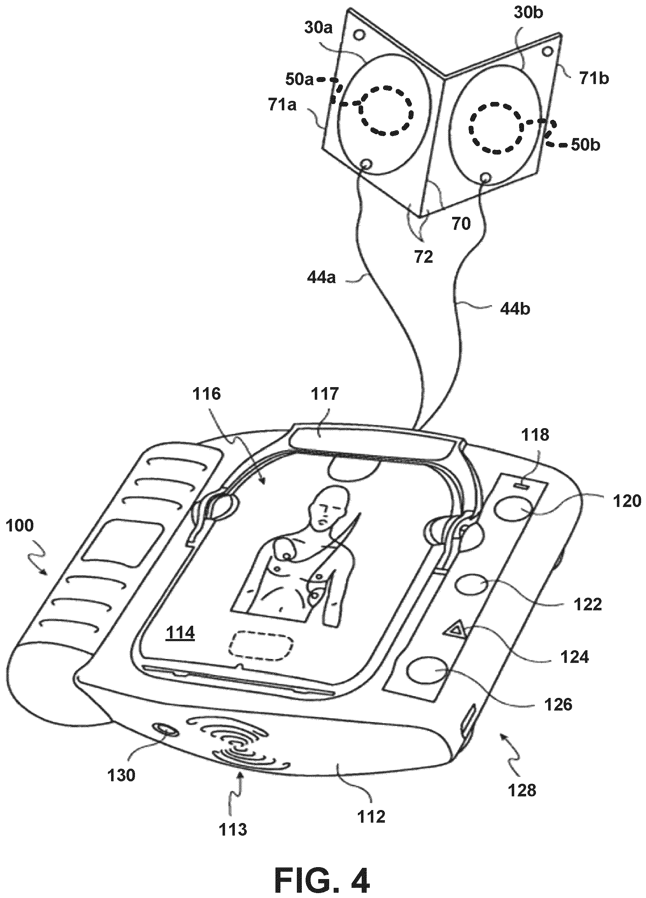

FIG. 4 illustrates defibrillator electrodes of the present disclosure coupled to a defibrillator 110. Defibrillator 110 represents a semi-automatic external defibrillator (AED). However, other types of external defibrillators can be used as well. The AED 110 is housed in a rugged polymeric case 112 which protects the electronic circuitry inside the case, and also protects the rescuer from shocks. Attached to the case 112 by electrical leads 44a,44b are a pair of defibrillator electrodes 30a,30b attached to the two sides 70a,70b of a release liner (not shown). Prior to use, the defibrillator electrodes 30a,30b are retained via a release liner 70 having two halves 71a, 71b secured by a hinge 72in a cartridge 114 located in a recess on the top side of the AED 110. The electrode pads are accessed for use by pulling up on a handle 117, which allows removal of the release liner 70. The user interface is on the right side of the AED 110. A small ready light 118 informs the rescuer of the readiness of the AED 110. In this embodiment the ready light blinks after the AED 110 has been properly set up and is ready for use. The ready light is on constantly when the AED 110 is in use, and the ready light is off or flashes in an alerting color when the AED 110 needs attention.

Below the ready light is an on/off button 120. The on/off button is pressed to turn on the AED 110 for use. To turn off the AED 110 the rescuer holds the on/off button down for one second or more. An information button 122 flashes when information is available for the rescuer. The rescuer depresses the information button to access the available information. A caution light 124 blinks when the AED 110 is acquiring heartbeat information from the patient and lights continuously when a shock is advised, alerting the rescuer and others that no one should be touching the patient during these times. A shock button 126 is depressed to deliver a shock after the AED 110 informs the rescuer that a shock is advised. An infrared port 128 on the side of the AED 110 is used to transfer data between the AED 110 and a computer. This data port finds used after the patient has been rescued and a physician desires to have the AED 110 event data downloaded to his or her computer for detailed analysis. A speaker 113 provides voice instructions to the rescuer to guide the rescuer through the use of the AED 110 to treat the patient. A beeper 130 is provided which "chirps" when the AED 110 needs attention such as electrode pad replacement or a new battery.

In accordance with the present disclosure, electrolyte bladders 50a,50b are adjoined to respective conductive electrode pads of defibrillator electrodes 30a,30b to facilitate electrically coupling the conductive electrode pads to a body of a patient wearing clothing or any other permeable material.

As shown in FIG. 4, in the exemplary embodiment, defibrillator electrodes 30a,30b are storable in a release liner 70. In practice, defibrillator electrodes 30a,30b can be stored in other case/cartridges as known in the art of the present disclosure or hereinafter conceived.

In practice, defibrillator 110 can monitor impedance between defibrillator electrodes 30a,30b so when the impedance changed this would indicate either an issue with exposure (e.g. a break in the skin seal of one or both electrolyte bladders) or that defibrillator electrodes 30a,30b had been deployed and attached to a patient. A sensor can also be included in case 70a and in communication with defibrillator 110 to detect the presence of moisture e.g., if the bladder had opened unintentionally. Again, an advantage of the electrolyte is that a thin clothing layer could remain on the patient because the electrolyte would penetrate and soak the clothing and provide an efficient transfer of energy for the ECG monitoring and electrotherapy via a defibrillation shock.

From the description of FIGS. 2-4 herein, those having ordinary skill in the art will appreciate the numerous benefits of the present disclosure including, but not limited to, a deployment of an electrolytic fluid as defibrillator electrodes are applied to a body of a patient. The fluid becomes the conductive pathway for electrocardiogram monitoring and electrical therapy. Indeed, as one having ordinary skill in the art would appreciate in view of the present disclosure, the present invention also can be used on patients without clothing on the their chest and/or torso.

The present disclosure has been described with reference to the preferred embodiments. Modifications and alterations may occur to others upon reading and understanding the preceding detailed description. It is intended that the invention be construed as including all such modifications and alterations insofar as they come within the scope of the appended claims or the equivalents thereof.

Moreover, all statements herein reciting principles, aspects, and exemplary embodiments of the present disclosure, as well as specific examples thereof, are intended to encompass both structural and functional equivalents thereof. Additionally, it is intended that such equivalents include both currently known equivalents as well as equivalents developed in the future (e.g., any elements developed that can perform the same or substantially similar functionality, regardless of structure). Thus, for example, it will be appreciated by one having ordinary skill in the art in view of the teachings provided herein that any block diagrams presented herein can represent conceptual views of illustrative system components and/or circuitry embodying the principles of the invention. Similarly, one having ordinary skill in the art should appreciate in view of the teachings provided herein that any flow charts, flow diagrams and the like can represent various processes which can be substantially represented in computer readable storage media and so executed by a computer, processor or other device with processing capabilities, whether or not such computer or processor is explicitly shown.

Having described preferred and exemplary embodiments of the present disclosure, which embodiments are intended to be illustrative and not limiting, it is noted that modifications and variations can be made by persons having ordinary skill in the art in view of the teachings provided herein, including the appended Figures and claims. It is therefore to be understood that changes can be made in/to the preferred and exemplary embodiments of the present disclosure which are within the scope of the present disclosure and exemplary embodiments disclosed, described and taught herein.

Moreover, it is contemplated that corresponding and/or related systems incorporating and/or implementing the device or such as can be used/implemented in a device in accordance with the present disclosure are also contemplated and considered to be within the scope of the present disclosure. Further, corresponding and/or related method for manufacturing and/or using a device and/or system in accordance with the present disclosure are also contemplated and considered to be within the scope of the present disclosure.

## Claims

1. A defibrillator electrode (30), comprising:
a conductive electrode pad (40), wherein, when the conductive electrode pad (40) is electrically coupled to a body of a patient and when the conductive electrode pad (40) is electrically coupled to an external defibrillator (60), the conductive electrode pad (40) is configured to transmit electrical signals from a heart of the patient to the external defibrillator (60) and to deliver a defibrillation shock from the external defibrillator (60) to the heart of the patient; and
an electrolyte bladder (50) adjoined to the conductive electrode pad (40), wherein, when the conductive electrode pad (40) is physically attached to a permeable material covering the body of the patient, the electrolyte bladder (50) is configured to release an electrolyte fluid to electrically couple the conductive electrode pad (40) to the body of the patient.

2. The defibrillator electrode (30) of claim 1,
wherein the conductive electrode pad (40) includes a gel layer configured to physically attach the conductive electrode pad (40) to the permeable material covering the body of the patient; and
wherein the electrolyte bladder (50) physically abuts the gel layer.

3. The defibrillator electrode (30) of claim 2,
wherein the conductive electrode pad (40) further includes an electrically conductive layer physically abutting the get layer diametric to the electrolyte bladder (50) physically abutting the gel layer.

4. The defibrillator electrode (30) of claim 2,
wherein a perimeter of a surface of the electrolyte bladder (50) physically abutting the gel layer is smaller than a perimeter of a surface of the gel layer physically attachable to the permeable material covering the body of the patient.

5. The defibrillator electrode (30) of claim 2, wherein the electrolyte bladder (50) and gel layer are concentrically aligned.

6. A defibrillation device, comprising:
an external defibrillator (60); and
a pair of defibrillator electrodes (30), each defibrillator electrode (30) includes:
a conductive electrode pad (40), wherein, when the conductive electrode pad (40) is electrically coupled to a body of a patient and when the conductive electrode pad (40) is electrically coupled to the external defibrillator (60), the conductive electrode pad (40) is configured to transmit electrical signals from a heart of the patient to the external defibrillator (60) and to deliver a defibrillation shock from the external defibrillator (60) to the heart of the patient; and
an electrolyte bladder (50) adjoined to the conductive electrode pad (40), wherein, when the conductive electrode pad (40) is physically attached to a permeable material covering the body of the patient, the electrolyte bladder (50) is configured to release an electrolyte fluid to electrically couple the conductive electrode pad (40) to the body of the patient.

7. The defibrillation device of claim 6,
wherein each conductive electrode pad (40) includes a gel layer configured to physically attach the respective conductive electrode pad (40) to the permeable material covering the body of the patient; and
wherein the electrolyte bladder (50) of the respective conductive electrode pad (40) physically abuts the gel layer of the respective conductive electrode pad (40).

8. The defibrillator device of claim 7,
wherein the conductive electrode pad (40) further includes an electrically conductive layer physically abutting the get layer diametric to the electrolyte bladder (50) physically abutting the gel layer.

9. The defibrillation device of claim 7,
wherein a perimeter of a surface of each electrolyte bladder (50) physically abutting the respective gel layer is smaller than a perimeter of a surface of the respective gel layer physically attachable to the permeable material covering the body of the patient.

10. The defibrillation device of claim 7,
wherein each electrolyte bladder (50) and the respective gel layer are concentrically aligned.

11. The defibrillation device of claim 6,
wherein, when the pair of defibrillator electrodes (30) are in storage, the pair of defibrillator electrodes (30) are configured to be electrically coupled together and the external defibrillator (60) is configured to test an impedance of the electrically coupled pair of defibrillator electrodes (30).

12. The defibrillation device of claim 11,
wherein, when the pair of defibrillator electrodes (30) are in storage, a change in impedance indicates at least one of a plurality of storage issues; and
wherein one of the plurality of the storage issues is a leakage of at least one of the electrolyte bladder (50)s.

13. The defibrillation device of claim 6, further comprising:
a sensor in electrical communication with the external defibrillator (60) and disposed adjacent one of the pair of defibrillator electrodes (30),
wherein, when the pair of defibrillator electrodes (30) are in storage, the sensor is configured to detect any leakage of the one of the pair of defibrillator electrodes (30).

14. A defibrillation method executable by a pair of defibrillator electrodes (30), each defibrillator electrode (30) including an electrolyte bladder (50) adjoined to a conductive electrode pad (40) electrically connected to an external defibrillator (60), the defibrillation method comprising;
releasing an electrolyte fluid from each electrolyte bladder (50) as the respective conductive electrode pad (40)s are being attached to permeable material covering a body of the patient to electrically couple the conductive electrode pad (40)s to the body of the patient; and
upon the conductive electrode pad (40)s being electrically coupled to the body of the patient by the electrolyte fluid, transmitting electrical signals from a heart of the patient through electrolyte fluid and the defibrillator electrodes (30) to the external defibrillator (60).

15. The defibrillation method of 14, further comprising:
subsequent to the transmitting electrical signals from the heart of the patient through the defibrillator electrodes (30) to defibrillator electrodes (30) to the external defibrillator (60), delivering a defibrillation shock from the external defibrillator (60) through the defibrillation electrodes and the electrolyte fluid to the heart of the patient.
